(19) Europäisches Patentamt
European Patent Office
Office européen des brevets

(11) **EP 4 767 987 A1**

(12) **EUROPEAN PATENT APPLICATION**
published in accordance with Art. 153(4) EPC

(43) Date of publication:
01.07.2026  Bulletin 2026/27

(51) International Patent Classification (IPC):
*A61B 34/37* (2016.01)

(21) Application number: 24867260.2

(86) International application number:
PCT/CN2024/115380

(22) Date of filing: 29.08.2024

(87) International publication number:
WO 2025/060846 (27.03.2025 Gazette 2025/13)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 19.09.2023  CN 202311214615
09.07.2024  CN 202410918476

(71) Applicant: Cornerstone Technology (Shenzhen)
Limited
Shenzhen, Guandong 518066 (CN)

(72) Inventors:
• WU, Jiahao
  Shenzhen, Guangdong 518066 (CN)
• WANG, Zerui
  Shenzhen, Guangdong 518066 (CN)
• AU, Kwok Wai Samuel
  Shenzhen, Guangdong 518066 (CN)

(74) Representative: Balder IP Law, S.L.
Paseo de la Castellana 93
5ª planta
28046 Madrid (ES)

(54) **SURGICAL ROBOT SYSTEM AND CONTROL METHOD THEREFOR**

(57)    The present disclosure provides a surgical robot system and a control method thereof. The surgical robot system includes: a first slave operation device including a first robotic arm, a second slave operation device including a second robotic arm, and a master operation device communicatively coupled to the first and second slave operation devices. The master operation device includes at least one first operation assembly configured to receive interactive operations from a user to control the first robotic arm or the second robotic arm. A respective first operation assembly is operable in a first operation mode and a second operation mode, in the first operation mode, the respective first operation assembly is configured to control operation of the first robotic arm, and in the second operation mode, the respective first operation assembly is configured to control operation of the second robotic arm.

FIG. 2

## Description

### CROSS REFERENCE TO RELATED APPLICATIONS

The present application claims priority to Chinese patent application No. 202311214615.X, entitled "SURGICAL ROBOT SYSTEM AND CONTROL METHOD THEREOF," filed on September 19, 2023, and Chinese patent application No. 202410918476.7, entitled "SURGICAL ROBOT SYSTEM AND CONTROL METHOD THEREOF," filed on July 9, 2024, each of which is incorporated by reference herein in its entirety.

### TECHNICAL FIELD

The present disclosure relates to the field of medical apparatus technology, and in particular to a surgical robot system and a method for controlling a surgical robot system.

### BACKGROUND

With the advancement of surgical technology, surgical robots are increasingly serving as valuable assistants to surgeons during procedures. Laparoscopic robot systems, in particular, offer high-precision operational capabilities and high-resolution visual feedback. This enables surgeons to observe the anatomical structure and details of the surgery site with exceptional clarity and perform operations with enhanced precision. Consequently, such systems contribute to significant reductions in intraoperative bleeding and shortened surgical durations for patients.

### SUMMARY

In a first aspect, the present disclosure provides a surgical robot system, including: a first slave operation device including a first robotic arm, one or more second slave operation devices including one or more second robotic arms, and a master operation device communicatively coupled to the first slave operation device and the one or more second slave operation devices. The master operation device includes at least one first operation assembly configured to receive interactive operations from a user to control the first robotic arm or the one or more second robotic arms. A respective first operation assembly of the at least one first operation assembly is operable in a first operation mode and a second operation mode, in the first operation mode of the respective first operation assembly, the respective first operation assembly is configured to control operation of the first robotic arm, and in the second operation mode of the respective first operation assembly, the respective first operation assembly is configured to control operation of the one or more second robotic arms.

In a second aspect, the present disclosure provides a method for controlling a surgical robot system.

The surgical robot system includes a first slave operation device including a first robotic arm, one or more second slave operation devices including one or more second robotic arms, and a master operation device communicatively coupled to the first slave operation device and the one or more second slave operation devices. The master operation device includes at least one first operation assembly. The method includes: receiving interactive operations from a user; and controlling a respective first operation assembly of the at least one first operation assembly to operate in an operation mode of a plurality of operation modes in response to the interactive operations. The plurality of operation modes include a first operation mode and a second operation mode, operation of the first robotic arm is controlled in the first operation mode, and operation of the one or more second robotic arms is controlled in the second operation mode.

In a third aspect, the present disclosure provides a surgical robot system, including: a plurality of slave operation devices, and a master operation device communicatively coupled to the plurality of slave operation devices. The master operation device is configured to control one or more of the plurality of slave operation devices concurrently.

Details of one or more embodiments of the present disclosure are presented in the accompanying drawings and description below. The other features, objectives, and advantages of the present disclosure will become apparent from the description, drawings, and claims.

### BRIEF DESCRIPTION OF THE DRAWINGS

The accompanying drawings are incorporated into the description and form a part of the description. In the accompanying drawings, embodiments of the present disclosure are illustrated and used together with the description to explain the principles of the present disclosure.

FIG. 1 is a schematic diagram of a structure of a master operation device of a surgical robot system according to some embodiments of the present disclosure;

FIG. 2 is a schematic diagram of a surgical operation scenario for slave operation devices according to some embodiments of the present disclosure;

FIG. 3 is a schematic diagram of another surgical operation scenario for the slave operation devices according to some embodiments of the present disclosure;

FIG. 4 is another schematic diagram of a surgical operation scenario for the slave operation devices according to some embodiments of the present disclosure;

FIG. 5 is a schematic diagram of a second operation assembly of the surgical robot system according to some embodiments of the present disclosure;

FIG. 6 is a schematic diagram of another second operation assembly of the surgical robot system according to some embodiments of the present disclosure;

FIG. 7 shows an application scenario of hysterectomy surgery using the surgical robot system according to the present disclosure;

FIG. 8 is a schematic diagram of a reference coordinate system fixed to a surgical instrument and a pose of the surgical instrument of the surgical robot system according to the present disclosure;

FIG. 9 is a schematic diagram of an eye of a surgeon, a reference coordinate system fixed to a master controller, and a pose of the master controller of the surgical robot system according to the present disclosure;

FIG. 10 is a schematic diagram of an interface in a display device of the surgical robot system according to the present disclosure;

FIG. 11 is a flowchart of a method for controlling a surgical robot system according to some embodiments of the present disclosure; and

FIG. 12 is a flowchart of the operation S200 of the method shown in FIG. 11 according to some embodiments of the present disclosure.

## DETAILED DESCRIPTION OF THE EMBODIMENTS

In the following, exemplary embodiments will be described in detail, and the accompanying drawings show corresponding examples. When referring to the accompanying drawings, unless otherwise indicated, the same numerals in different drawings represent the same or similar elements. The exemplary embodiments described in the following do not represent all embodiments according to the present disclosure. They are only examples of devices and methods according to some claimed aspects of the present disclosure.

The terms used in the present disclosure are for the purpose of describing specific embodiments only and are not intended to limit the scope of the present disclosure. Unless otherwise defined, the technical or scientific terms used in the present disclosure shall have the usual meanings understood by those skilled in the art to which the present disclosure belongs. The use of "first", "second", and the like in the description and claims of the present disclosure does not indicate any order, quantity, or importance, but is only used to distinguish different components. Similarly, wordings like "a" or "an" do not indicate a quantity limit, but rather indicate the existence of at least one. "Multiple" or "a plurality of" indicates the existence of at least two. Unless otherwise specified, terms such as "front", "rear", "lower", and/or "upper" are only for ease of illustration and are not limited to a specific location or spatial orientation. Wordings such as "comprising" or "including" refer to that the elements or objects before "comprising" or "including" include those and their equivalents listed after "comprising" or "including", and do not exclude other elements or objects. Wordings like "connection" or "coupling" are not limited to physical or mechanical connections, and can include electrical connections, whether direct or indirect.

The singular forms of "a", "said", and "the" used in the description and the accompanying claims of the present disclosure are also intended to include the plural form, unless the context clearly indicates otherwise. It should also be understood that the term "and/or" used in the present disclosure refers to and includes any or all possible combinations of one or more listed items associated with each other.

A surgical robot system is a robot that can be remotely manipulated to perform surgery, and usually includes a master operation device (also known as a surgeon console), a slave operation device (also known as a patient-side robot), and an imaging system.

The control system has a display unit for displaying the environment for surgical instruments, a surgeon-operated control mechanism, and an armrest. The display unit is equipped with an observation window for the surgeon to observe, the control mechanism is such designed that the manipulation of the control mechanism effects the operation of surgical instruments, and the armrest is used to support the surgeon's arm. In addition, there are other control switches on the surgeon console that are convenient to be touched or pressed by hands or feet to perform various functional operations for human-apparatus interaction.

The imaging system has a display screen, an endoscope controller, system electronics, an image processor, and the like.

The patient-side robot, such as a laparoscopic robot, typically includes at least one robotic arm that can manipulate multiple surgical instruments. By using complex algorithms, the robotic arm simulates the movement of a surgeon's arm and provides high-precision and accurate movement control.

The surgical robot system can provide high-precision surgical manipulation and surgical view, but for some complex surgical operations, additional surgeons or surgical assistants are required for assistance to the operations. Taking gynecological surgery as an example, an assistant is needed to manipulate a uterine manipulator to move or orientate the uterus, in order to provide the surgeon with a good surgical view. To this end, an auxiliary manipulation robot such as a endoscope-holding robot and a uterine-manipulating robot is introduced into the surgical robot system, which is commonly used for auxiliary operations in traditional laparoscopic surgeries.

In some scenarios, the assistant manually operates the auxiliary manipulation robot. The assistant needs to manually move an arm of the auxiliary manipulation robot to adjust the position and pose thereof. In this way, the surgeon's intention can be conveyed through real-time feedback from the assistant, but it requires multiple communications and confirmations between the surgeon and the assistant, which is inefficient and prone to misunderstandings or errors, leading to increase in uncertainty during the surgery.

Thus, the present disclosure provides a surgical robot system, in order to overcome or alleviate one or more of the aforementioned drawbacks.

The surgical robot system according to the embodiments of the present disclosure may include a master operation device and a plurality of slave operation devices. The master operation device is communicatively coupled to the plurality of slave operation devices, such that the master operation device can control one or more of the plurality of slave operation devices concurrently. The plurality of slave operation devices may include at least two slave operation devices, which may be for example two or more of a single-port patient-side operation device, a single-arm patient-side operation device, a multi-port patient-side operation device, a flexible patient-side operation device, and the like.

The surgical robot system may be switchable to one of a plurality of control modes including at least one exclusive control mode and at least one non-exclusive control mode. In an exclusive control mode, the system is configured to exclusively control one slave operation device of the plurality of slave operation devices, and in a non-exclusive control mode, the system is configured to control merely one slave operation device or control at least two slave operation devices concurrently, depending on the complexity of the surgery or special requirements.

The system may select a default control mode upon access of a slave operation device. For example, the system matches a type of the accessed slave operation device with pre-stored information in the system to select a corresponding control mode. Alternatively, the access of the slave operation device to the system may trigger an interactive interface of the master operation device, through which the surgeon may select a desired control mode.

The surgeon also may manually switch control modes according to surgical operation requirements. The switching operations may be preform through, but not limited to, specific actions on master controllers of the master operation device, buttons or sensing devices on the master controller, physical triggering devices such as pedals or sensing triggering devices of the master operation device, interactive operations on the display interface of the master operation device (such as operations on the display interface overlaid on a surgical scene via the master controller functioning as a mouse, or operations on a touch screen provided on the armrest), or a combination of the above operations configured logically. In addition, the system may automatically determine an appropriate control mode based on specific recorded information during the surgery and remind the surgeon through, for example, the text reminders on the display interface overlaid on the surgical scene or the voice broadcast reminders.

In some embodiments, the master operation device includes two first operation assemblies, each of the two first operation assemblies is configured to receive interactive operations from a user to control operation of a respective slave operation device of the plurality of slave operation devices.

In an exclusive control mode of the surgical robot system, each of the two first operation assemblies is configured to control operation of an instrument selected from instruments in a same slave operation device, or control two instruments selected from instruments in a same slave operation device, respectively.

In a non-exclusive control mode of the surgical robot system, each first operation assembly may be configured to control operation of an instrument selected from instruments in the plurality of slave operation devices. The two first operation assemblies may control a same instrument, or control two instruments, respectively. The two instruments may be held by a same slave operation device, or be held by two slave operation devices, respectively.

For example, the two first operation assemblies may be devices for the surgeon to operate with left and right hands respectively. In a non-exclusive control mode, the object controlled by each respective first operation assembly may be freely selected from the instruments held by the plurality of slave operation devices. For example, the system includes a first slave operation device and a second slave operation device. The surgeon may manipulate an instrument in the first slave operation device with left hand and manipulate an instrument in the second slave operation device with right hand, or may manipulate an instrument in the first slave operation device or in the second slave operation device with both left and right hands, or may operate two different instruments in either the first slave operation device or the second slave operation device with left and right hands, respectively.

In some embodiments, in a non-exclusive control mode, further restrictions or constraints may be imposed based on the surgeon's guidance and teaching needs or special surgical scenes, such as the two first operation assemblies are commanded to control the instruments in different slave operation devices separately.

In some embodiments, in a non-exclusive control mode, possible faults may include: cross-system faults between multiple surgical robots and individual system faults of the surgical robots, depending on where the faults occur; and recoverable faults and non-recoverable faults, depending on the nature of the fault. The master operation device can control the states using existing mature technologies of robots to deal with the faults, in order to ensure the safety of patients during surgery.

In some embodiments, the plurality of slave operation devices may include a single-port laparoscopic robot and at least one single-arm auxiliary robot.

A single-port surgical robot has advantages such as fewer incisions and easier deploy. A single-port surgical robot typically includes a multi-degree-of-freedom endoscope and multiple surgical instruments which pass through a cannula when installed. The surgical instruments for the single-port laparoscopic robot typically each have six to seven degrees of freedom for the operation of an end effector of the surgical instrument, these degrees of freedom are mainly achieved by the elbow joints for positional movements and the wrist joints for orientation changes. Due to the large number of joints and complex structure of the surgical instruments, their output force and stiffness are often limited, making them inadequate for applications requiring higher output forces. In addition, due to the high complexity, instruments such as ultrasonic scalpels, vascular sealers, and staplers suitable for the single-port laparoscopic robot cannot meet some surgical operation requirements.

To adapt to some specific surgical procedures or complex surgical scenes, and improve the flexibility and convenience of surgical operations, a single-arm auxiliary robot may be introduced before or during the surgery. The single-arm auxiliary robot may be equipped with instruments suitable for the multi-port laparoscopic robot to achieve greater output force and be compatible with instruments such as ultrasonic scalpels, vascular sealers, staplers, and the like, thereby meeting a wider range of clinical needs.

In these application scenarios, the system may be switchable to one of three control modes: a single-port control mode, a single-arm control mode, and a single-port-single-arm non-exclusive control mode.

In the single-port control mode, the master operation device is configured to control exclusively a single-port laparoscopic robot. The master operation device may control the single-port laparoscopic robot to manipulate the surgical instruments, such as controlling the mechanical arm of the single-port laparoscopic robot to move the surgical instruments, or controlling the wrists or end effectors of the surgical instruments to perform operations. In this control mode, the system may also switch between preset sub-modes that match the corresponding surgical scenes according to the states of the cannula, endoscope, and surgical instruments, such as a preset mode for adjusting the posture of the endoscope to have the optimal surgical view, or a preset mode for retracting instruments.

In the single-arm control mode, the master operation device is configured to control only a single-arm auxiliary robot. The single-arm auxiliary robot may be equipped with a unique endoscope for the entire system or an additional endoscope, a uterine manipulator, an energy instrument such as an ultrasonic scalpel, and an advanced instrument such as a stapler. The master operation device may control the single-arm auxiliary robot to manipulate the surgical instrument, such as controlling the mechanical arm of the single-arm auxiliary robot to move the surgical instrument. It is noted that in the single-arm control mode, the single-arm auxiliary robot may also cooperate with traditional manual instruments for complementary surgical operations.

In the single-port-single-arm non-exclusive control mode, the master operation device may be configured to control only a single-port laparoscopic robot, only a single-arm auxiliary robot, or both the single-port laparoscopic robot and the single-arm auxiliary robot. Each first operation assembly of the master operation device may freely select a respective instrument from the instruments of both the single-port laparoscopic robot and the single-arm auxiliary robot for control. For example, one of the first operation assemblies may be configured to control a certain instrument of the single-port laparoscopic robot, while the other first operation assembly may be configured to control an instrument of the single-arm auxiliary robot. Alternatively, both the two first operation assemblies may be configured to jointly control the same instrument of the single-port laparoscopic robot, or the two first operation assemblies may control two different instruments of the single-port laparoscopic robot, respectively. Alternatively, both the two first operation assemblies may jointly control an instrument of the single-arm auxiliary robot.

In some other embodiments, the plurality of slave operation devices may include a multi-port laparoscopic robot and at least one single-arm auxiliary robot. In this case, the system may be switchable to one of a multi-port control mode, a single-arm control mode, and a multi-port-single-arm non-exclusive control mode. How the system works under these control modes and how the system switches between the control modes are similar to those illustrated in above embodiments, and will not be repeated here.

The selection and switching process of the control modes of the surgical robot system is as follows. When the system starts, a corresponding control mode is automatically selected according to the types of the robots. For example, when both a single-port laparoscopic robot and a single-arm auxiliary robot are activated, the single-port-single-arm non-exclusive control mode is selected. During the operation of the system, upon interactive operations being detected, the function of switching control modes is activated, and when no interactive operation is detected, the current control mode is maintained. The interactive operations may include, for example, stepping on a pedal or pressing a button on the master controller. When the function of switching control modes is activated, a surgeon may switch control modes according to surgical needs. For example, the surgeon may manually select through specific interactive physical operations on the master operation device or the interactive interface displayed on the display device of the master operation device. For example, the interactive interface may be overlaid on the visual display of the surgical scene, and the surgeon may manipulate the master controller to scroll through or slide across the options in the interactive interface. Upon navigating to a desired control mode, the selection may be confirmed via a double-click of a button on the master controller or through other physically interactive mechanisms, thereby accomplishing the switching of the control mode.

Some embodiments of the present disclosure provide a control method, which relates to switching control of multiple slave operation devices of a surgical robot system in a non-exclusive control mode.

In the following, the surgical robot system and the control method therefor according to the present disclosure will be described in detail with reference to the accompanying drawings. The features in the following embodiments may be combined with each other as long as no contradiction occurs.

The surgical robot system provided in some embodiments of the present disclosure includes: a first slave operation device including a first robotic arm for holding at least one instrument, a second slave operation device including a second robotic arm for holding at least one instrument, and a master operation device. The instruments may be surgical instruments or endoscopes. For the first robotic arm and/or second robotic arm, the movement of the robotic arms can move the instruments in at least two degrees of freedom, such as pitching and yawing. In addition, a drive assembly is provided at the robotic arm and coupled to the instrument to drive the instrument to insert and rotate, or to drive the wrist of the instrument for pitching and yawing movements, or to drive the end effector of the instrument to open and close. The master operation device is communicatively coupled to the first slave operation device and the second slave operation device. The surgeon works at the master operation device and controls the first and/or second slave operation devices to perform surgery by manipulating the master operation device.

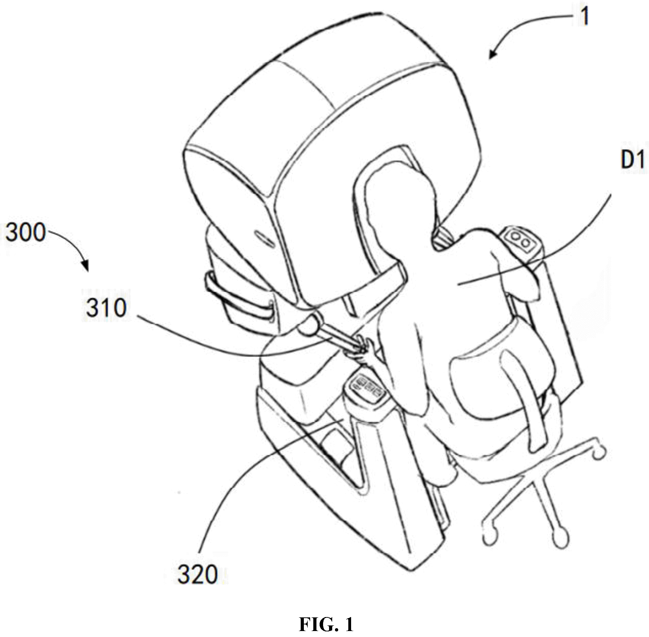

FIG. 1 is a schematic diagram of a structure of a master operation device of a surgical robot system according to some embodiments of the present disclosure. The master operation device 300 includes at least one first operation assembly 310 configured to receive interactive operations from a user. The surgeon D1 may interact with the master operation device 300 through the at least one first operation assembly 310. Upon receiving the interactive operation from the user, the master operation device 300 generates corresponding operation instructions, and the first and/or second slave operation devices perform corresponding actions under command of the operation instructions, such as manipulating the first robotic arm and/or the second robotic arm. The operation mode of each first operation assembly 310 includes a first operation mode and a second operation mode associating with the control of the first robotic arm and the second robotic arm, respectively.

Manipulation of the first robotic arm and/or the second robotic arm may include driving the entire instrument to pitch, yaw, or the like through the movements of the robotic arm, or driving the instrument to insert and rotate via a drive assembly provided on the robotic arm, or driving the wrist of the instrument to pitch, yaw, or the like via the drive assembly, or driving the end effector of the instrument to open and close via the drive assembly.

In the first operation mode of a respective first operation assembly 310, the respective first operation assembly is configured to control operation of the first robotic arm. For example, when a first operation assembly 310 is in the first operation mode, the master operation device 300 generates corresponding operation instructions based on the interactive operations received by the first operation assembly 310, and sends the operation instructions to a first slave operation device. The first slave operation device manipulates the first robotic arm according to the operation instructions.

In the second operation mode of a respective first operation assembly 310, the respective first operation assembly is configured to control operation of the second robotic arm. For example, when a first operation assembly 310 is in the second operation mode, the master operation device 300 generates corresponding operation instructions based on the interactive operations received by the first operation assembly 310, and sends the operation instructions to a second slave operation device. The second slave operation device manipulates the second robotic arm according to the operation instructions.

Generally, as shown in FIG. 1, The surgeon D1 may control the movement of surgical instruments held on the first and second robotic arms by operating the first operation assembly 310. According to the embodiments of the present disclosure, the surgeon may control the first and second slave operation devices through the master operation device 300 to achieve more convenient switching between the first and second slave operation devices, without the need for surgical assistants or additional auxiliary equipment for control, thereby simplifying the operation process, lowering the surgeon's learning curve, and increasing surgical flexibility and maneuverability.

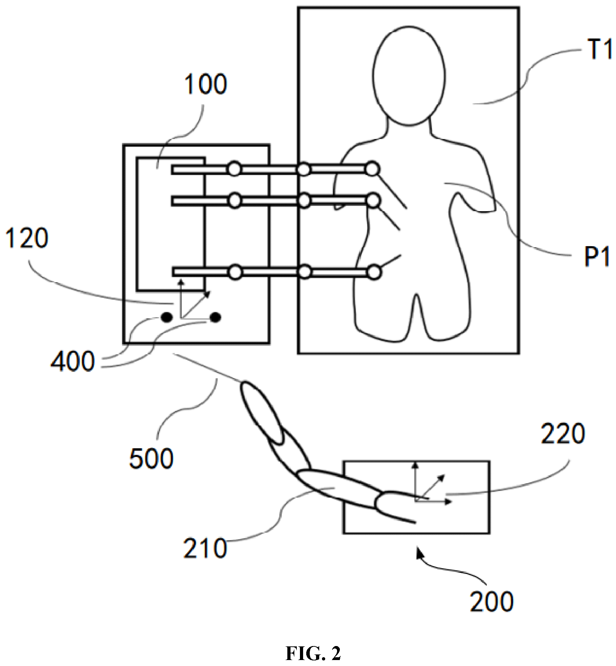

FIG. 2 to FIG. 4 illustrate some application scenarios of the slave operation devices. As shown in the drawings, T1 represents an operating table, and P1 represents the patient on the operating table T1. In some embodiments, the first slave operation device 100 includes a laparoscopic robot having one or at least two first robotic arms 110. The second slave operation device 200 includes an auxiliary manipulation robot having one or at least two second robotic arms 210. It is noted that the auxiliary manipulation robot is a robot used for auxiliary operations in addition to the main operations of the laparoscopic robot, and may usually be used to perform some relatively simple surgical operations or auxiliary operations. The position where the auxiliary manipulation robot enters the body of the patient P1 can be more flexible, that is, it can enter the body of the patient P1 from various positions for surgical operations, such as entering through the natural channel (such as vagina or urethra) of the patient P1. Therefore, the auxiliary manipulation robot can provide additional surgical approach angles and enhanced maneuverability.

Generally, to enhance the convenience and practicality of the auxiliary manipulation robot, it may be deployed as a standalone unit, for example, the auxiliary manipulation robot may not share a common base with the main body of the laparoscopic robot. In this way, the surgeon and operating room assistants are more convenient in handling intraoperative issues, without interference with other surgical operations. When used in conjunction with the laparoscopic robot, the auxiliary manipulation robot can improve surgical efficiency and safety, and reduce the burden on the surgeon and operating room assistants.

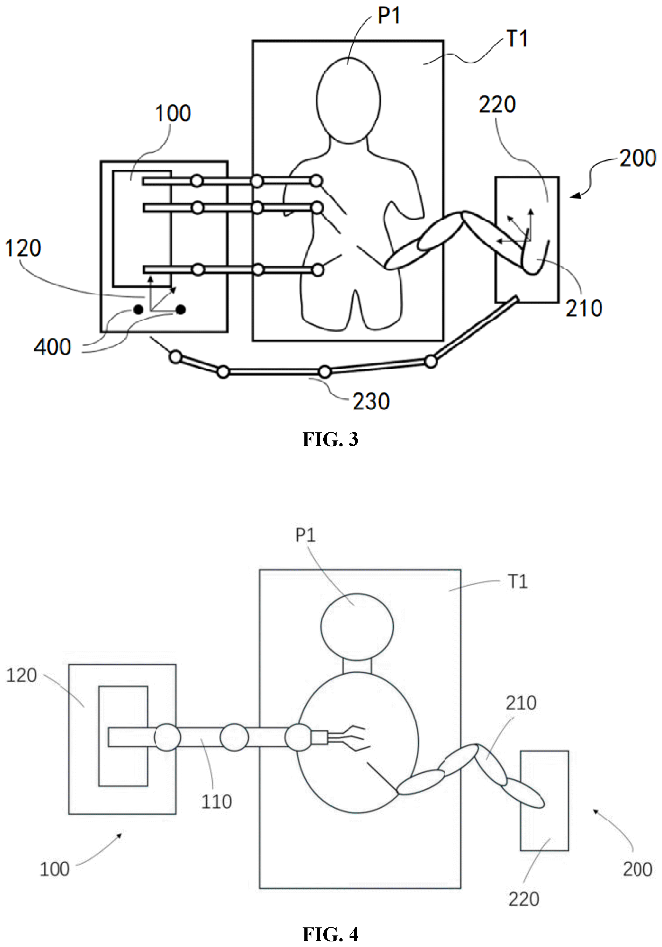

In the examples shown in FIGS. 2 and 3, the first slave operation device 100 is a multi-port laparoscopic robot including at least two first robot arms 110, for example three or four first robot arms. Each first robot arm 110 holds a respective surgical instrument or endoscope and enters the human abdominal cavity through a respective abdominal access port for surgical operations. When a first operation assembly 310 is in the first operation mode, the master operation device 300 generates corresponding operation instructions based on the interactive operations received by the first operation assembly 310, and sends the operation instructions to the first slave operation device. The first slave operation device manipulates one of the first robotic arms according to the operation instructions.

In the example shown in FIG. 4, the first slave operation device 100 is a single-port laparoscopic robot including a first robot arm 110, and the first robot arm 110 holds at least two instruments, for example three or four instruments, and for example an endoscope and at least one surgical instrument. The at least two instruments are controlled to enter the human abdominal cavity through a same abdominal access port for surgical operations. Each instrument is coupled to a respective drive assembly provided on the first robotic arm 110, and each drive assembly is used to drive the respective instrument. When a first operation assembly 310 is in the first operation mode, the master operation device 300 generates corresponding operation instructions based on the interactive operations received by the first operation assembly 310, and sends the operation instructions to the first slave operation device. The first slave operation device manipulates the first robotic arm according to the operation instructions. In this case, one of the drive assemblies on the first robotic arm is activated to manipulate the respective instrument coupled thereto according to the operation instructions.

In the examples shown in FIGS. 2 to 4, the second slave operation device 200 is a single-arm auxiliary manipulation robot including a second robot arm 210, and the second robot arm 210 holds a surgical instrument or an endoscope. For example, in the application scenario shown in FIG. 2, the second robotic arm 210 may hold a uterine manipulator and enter the uterus through the human vagina to move the uterus. For example, in the application scenarios shown in FIGS. 3 and 4, the second robotic arm 210 may hold an endoscope and enter the human abdominal cavity through an abdominal access port for capturing images. This abdominal access port is usually different from the abdominal access port for the surgical instrument or endoscope held by the first robotic arm 110.

Moreover, the first operation assembly 310 of the master operation device 300 may include a master controller. The master controller may be provided on the console, and the surgeon may control the laparoscopic robot and the auxiliary manipulation robot by manipulating the master controller. The master controller may include a gripping end, and the surgeon may hold and operate on the gripping end with a hand. The master controller may further include at least two joints, and the gripping end is movably coupled to the master operation device 300 through the joints, thereby providing one or more degree of freedom of movement for the gripping end. In an application scenario, in the first operation mode, the surgeon holds and operates on the gripping end of the master controller, and the operation of the surgeon on the master controller is mapped to the manipulation of the first robotic arm 110. In the second operation mode, the surgeon holds and operates on the gripping end of the master controller, and the operation of the surgeon on the master controller is mapped to the manipulation of the second robotic arm 210. Each of the first robotic arm 110 and the second robotic arm 210 has multiple degrees of freedom. The surgeon may manipulate the master controller to change the angles and orientations of the first robotic arm 110 and the second robotic arm 210, thereby moving the surgical instruments, such as to rotate about a preset remote center of motion (RCM).

It should be noted that the present disclosure does not limit types of the mechanisms for achieving the rotation of the first robotic arm 110 and the second robotic arm 210 about the RCM. The mechanisms may include mechanically implemented mechanisms and/or software implemented mechanisms. The RCM is set at the optimal physiological position for the corresponding task, such as on the cannula inserted into the abdominal cavity, or the vaginal orifice or the cervical orifice for uterine manipulation.

It is noted that there may be one or two master controllers.

In a case where there is one master controller, the surgeon may manipulate the master controller to control either the first robotic arm 110 or the second robotic arm 210.

In a case where there are two master controllers, namely a left-hand master controller and a right-hand master controller, the surgeon may manipulate the two master controllers to control the first robotic arm 110. In a case where there are two or more first robotic arms 110, the surgeon may manipulate the two master controllers to control two different first robotic arms 110, respectively. The surgeon may manipulate the two master controllers to control the second robotic arm 210. In a case where there are two or more second robotic arms 210, the surgeon may manipulate the two master controllers to control two different second robotic arms 210, respectively. The surgeon may manipulate one of the two master controllers to control a first robotic arm 110, and manipulate the other master controller to control a second robotic arm 210. The corresponding relationships of control may be adjusted according to the actual situation to facilitate the operation of the surgeon, and will not be limited in the present disclosure.

It is noted that in the application scenario where two master controllers are used to control a same robotic arm simultaneously, it is necessary to fix the distance between the tip coordinate systems of the two master controllers to prevent changes in the distance between coordinate systems from causing inconvenience to the surgeon during operation.

It can be understood that, as illustrated above, the auxiliary manipulation robot has a base separated from that of the main body of the laparoscopic robot. Therefore, prior to a surgery, it is necessary to calibrate the positions and orientations of the bases of the auxiliary manipulation robot and of the main body of the laparoscopic robot. That is, calibration of the relative positional relationship between the coordinate system 220 of the base of the auxiliary manipulation robot and the coordinate system 120 of the base of the laparoscopic robot is required. The base of the auxiliary manipulation robot and the base of the main body of the laparoscopic robot may be cart-based bases or ceiling-suspended bases.

In some embodiments, taking FIG. 2 as an example, two markers 400 are set on the base of main body of the laparoscopic robot. Of course, the two markers 400 may also be set on other parts fixed relative to the base of main body of the laparoscopic robot. For example, a marker 400 may be a solid ball, a concave dot, or a needle tip. It is noted that the positions of the two markers 400 in the coordinate system 220 of the base of the auxiliary manipulation robot are known.

The calibration process is as follows: the operator may move the tips of the surgical instruments 500 of the auxiliary manipulation robot to touch the two markers 400 in sequence, and resolve the position information of the two markers 400 in the coordinate system 220 of the base of the auxiliary manipulation robot based on the kinematic data of the robot through calculation. The relative positional relationship between the two coordinate systems is calculated based on the position information of the two markers 400 in the coordinate system 220 of the base of the auxiliary manipulation robot and in the coordinate system 120 of the base of the laparoscopic robot.

The proximal end of the first robotic arm 110 is connected to the base of the first slave operation device 100, and the distal end may hold the surgical instrument 500. Multiple joints may be provided between the proximal and distal ends of the first robotic arm 110, thereby providing one or more degrees of freedom for the distal end. Encoders are provided at the joints to detect the rotation angles of the joints. The proximal end of the second robotic arm 210 is connected to the base of the second slave operation device 200, and the distal end may hold the surgical instrument 500. Multiple joints may be provided between the proximal and distal ends of the second robotic arm 210, thereby providing one or more degrees of freedom for the distal end. Encoders are provided at the joints to detect the rotation angles of the joints. The surgical instruments 500 may include scissors, clamps, and puncture needles for performing invasive procedures on human tissue, and/or include imaging devices such as endoscopes for capturing surgical images, and/or tools for moving organs for surgical operations.

It is noted that the terms "distal end" and "proximal end" used in the present disclosure are directional words, where "distal end" refers to the end far from the base, and "proximal end" refers to the end close to the base.

In some other embodiments, taking FIG. 3 as an example, in a case where the distance between the bases of two robots is relatively large, an auxiliary registration arm 230 provided on the auxiliary manipulation robot may be used. Encoders are provided at the joints of the auxiliary registration arm 230. In this way, the position information of the two markers 400 in the coordinate system 220 of the base of the auxiliary manipulation robot may be resolved based on the kinematic data of the auxiliary registration arm 230.

As an alternative to the embodiments shown in FIGS. 2 and 3, the markers 400 may also be set on the base of the auxiliary manipulation robot or other parts fixed relative to the base of the auxiliary manipulation robot, and the tip of the robotic arm of the laparoscopic robot may be moved to touch the markers 400. The markers 400 may also be set at other positions fixed relative to the ground, for example, on the operating table T1, and the robotic arm of the laparoscopic robot and the robotic arm of the auxiliary manipulation robot may be moved to touch the markers 400 using their tips.

Referring to FIG. 1, in some embodiments, the master operation device 300 may further include a second operation assembly 320 configured to receive interactive operations from the user to switch an operation mode of the first operation assembly 310. It can be understood that the second operation assembly 320 may serve as an input assembly for the surgeon's control instructions, and the second operation assembly 320 may receive control instructions input by the surgeon through appropriate interaction methods. The interaction methods may include voice control input, display control input, headset equipment input, eye movement trajectory input, finger button input, foot pedal input, and other interaction methods. The interaction methods do not require the surgeon to move hands away from the master controller, and allows the surgeon's head to remain within the observation window of the display unit to maintain observation of the lesion area, thereby reducing the complexity of operation for the surgeon and ensuring the safety of surgery. The interaction methods may also be integrated into the master controller, for example, there is a button on the master controller. Upon the surgeon pressing the button, the system switches the operation mode of the first operation assembly 310 directly, or activates the voice control input mode or display control input mode where switch of the operation mode of the first operation assembly 310 is available with the surgeon's voice commands or selection on a screen. For example, the surgeon may use headset equipment to manipulate the first robotic arm 110 or the second robotic arm 210 based on the posture of the surgeon's head. For example, the first robotic arm 110 or the second robotic arm 210 are manipulated based on the eye focus of the surgeon.

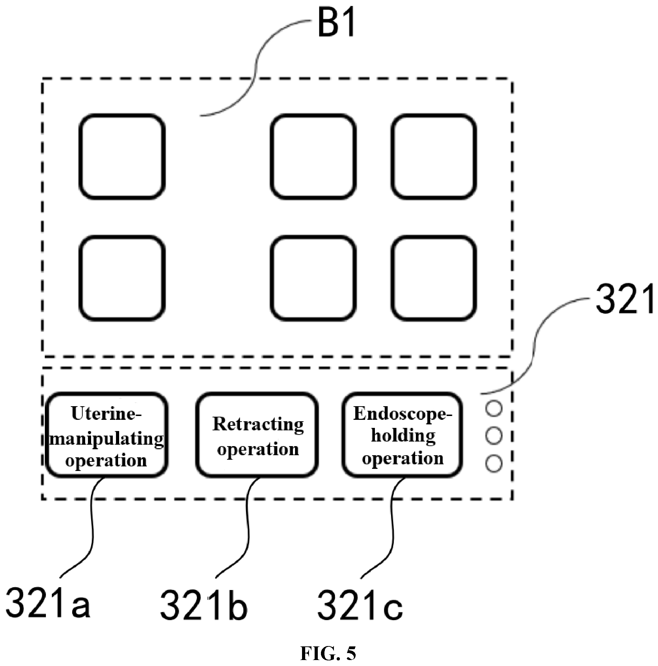

FIG. 5 is a schematic diagram of a second operation assembly 320 of the surgical robot system according to some embodiments of the present disclosure. As shown in FIG. 5, B1 represents some original pedals of the surgical robot system 1. In addition to the original pedals, the second operation assembly 320 includes at least one pedal 321 configured to detect stepping action from the user and generate a corresponding switching signal. The master operation device 300 is configured to switch the operation mode of the first operation assembly 310 according to the switching signal. The at least one pedal 321 may be implemented as a set of pedals 321 including at least one sub-pedal. As shown in FIG. 5, the pedal 321 may be provided close to the original pedal B1 to lower the surgeon's learning curve.

For example, in a case where there are two first operation assemblies (i.e. master controllers) 310, and there is one or a set of pedals 321, the surgeon may switch the operation modes of the two master controllers by stepping on the one or a set of pedals 321. For example, when the surgeon steps on one of the pedals 321, the operation modes of both master controllers may be switched concurrently; or the operation mode of one of the master controllers may be switched first, and then the operation mode of the other master controller may be switched by stepping on the pedal 321 again. The above control methods may be flexibly adjusted and are not limited in the present disclosure.

In some embodiments, the second slave operation device 200 includes at least two second robotic arms 210. In some other embodiments, the surgical robot system 1 includes at least two second slave operation devices 200, and each of them includes at least one second robotic arm 210. In still some other embodiments, the surgical robot system 1 includes at least two second slave operation devices 200, each of them includes at least one second robotic arm 210, and at least one of them includes at least two second robotic arms 210. That is, at least two second robotic arms 210 are provided for auxiliary operations.

Accordingly, in some embodiments, the second operation assembly 320 may include at least two pedals 321, and each of the at least two pedals 321 is operatively coupled to a respective second robotic arm 210 and is configured to detect the stepping operation from the user and to generate a respective switching signal. The master operation device 300 is configured to switch the operation mode of the first operation assembly 310 into the second operation mode in response to the respective switching signal, and the first operation assembly 310 is configured to control, in the second operation mode, operation of the respective second robotic arm 210 operatively coupled to a pedal 321. The at least two pedals 321 may be implemented as at least two sets of pedals 321, and each set of pedals 321 includes at least one sub-pedal.

In some application scenarios, for example, three second robotic arms 210 are provided for auxiliary operations and configured to hold different instruments, respectively, such as including a uterine manipulator, a retractor, and an endoscope. The three second robotic arms 210 may be respectively provided in three independent second slave operation devices 200, or provided in a same independent second slave operation device 200, or distributed in two independent second slave operation devices 200. Accordingly, pedals operatively coupled to the three second robotic arms 210 may be provided.

For example, as shown in FIG. 5, the pedals 321 may include a uterine-manipulating-operation pedal 321a operatively coupled to the second robotic arm 210 holding the uterine manipulator. Upon the surgeon stepping on the uterine-manipulating-operation pedal 321a, the uterine-manipulating-operation pedal 321a generates a uterine manipulator switching signal. The master operation device 300 is configured to receive the uterine manipulator switching signal and switch the first operation assembly 310 to the second operation mode. The first operation assembly 310 is configured to control operation of the second robotic arm 210 operatively coupled to the uterine-manipulating-operation pedal 321a, thereby achieving the movement of the uterine manipulator.

For example, as shown in FIG. 5, the pedals 321 may include a retracting-operation pedal 321b operatively coupled to the second robotic arm 210 holding the retractor. Upon the surgeon stepping on the retracting-operation pedal 321b, the retracting-operation pedal 321b generates a retractor switching signal. The master operation device 300 is configured to receive the retractor switching signal and switch the first operation assembly 310 to the second operation mode. The first operation assembly 310 is configured to control operation of the second robotic arm 210 operatively coupled to the retracting-operation pedal 321b, thereby achieving the movement of the retractor.

For example, as shown in FIG. 5, the pedals 321 may include an endoscope-holding-operation pedal 321c operatively coupled to the second robotic arm 210 holding the endoscope. Upon the surgeon stepping on the endoscope-holding-operation pedal 321c, the endoscope-holding-operation pedal 321c generates an endoscope switching signal. The master operation device 300 is configured to receive the endoscope switching signal and switch the first operation assembly 310 to the second operation mode. The first operation assembly 310 is configured to control operation of the second robotic arm 210 operatively coupled to the endoscope-holding-operation pedal 321c, thereby achieving the movement of the endoscope.

The types and numbers of the pedals 321 are not limited to those illustrated above, and may be determined according to the types of the surgical instruments 500 held by the second robotic arms 210.

In some embodiments, the second operation assembly 320 further includes a display device 322 configured to display a selection interface. The selection interface has at least two selection icons 323 to be selected by the user, and each of the at least two selection icons 323 corresponds to a respective second robotic arm 210. The first operation assembly 310 is further operable in a third operation mode, and in the third operation mode of the first operation assembly, the first operation assembly 310 is configured to control a cursor 324 in the selection interface to select a selection icon 323 and then confirm the selection. The master operation device 300 is configured to switch the operation mode of the first operation assembly 310 into the third operation mode in response to the switching signal and control the display device to display the selection interface, and further configured to switch the operation mode of the first operation assembly 310 into the second operation mode in response to selection on a selection icon 323 being confirmed. In the second operation mode, the first operation assembly 310 is configured to control operation of a second robotic arm 210 corresponding to the selection icon 323 being selected. In this way, in the third operation mode, the surgeon can select the second robotic arm 210 corresponding to the selection icon 323 on the screen, and to manipulate the selected second robotic arm 210, thereby facilitating intuitive and quick selection of the required instruments by the surgeon.

In addition, as the surgeon can intuitively perform the selection and confirmation on the display device, the second operation assembly 320 may include merely one pedal for auxiliary operations. Upon the surgeon stepping on the pedal, the operation mode of the first operation assembly 310 is switched to the third operation mode where the display device displays the selection interface to facilitate the selection operations and confirmation of the selection by the surgeon, thereby further lowering the learning curve of the surgeon.

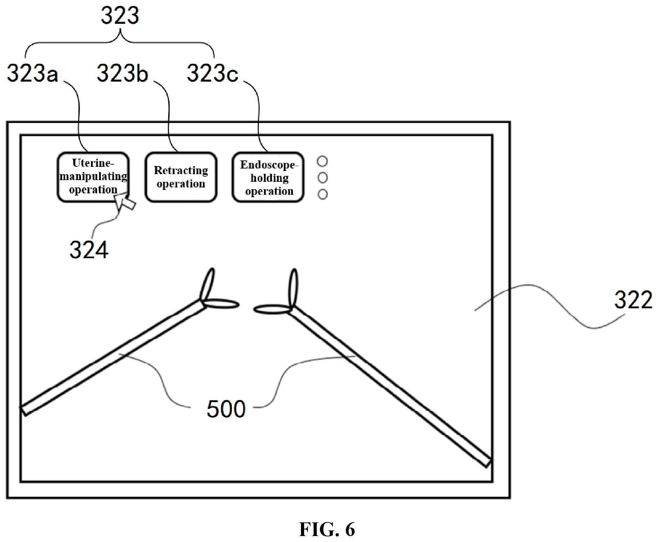

During a surgery, referring to FIG. 1, the surgeon usually views the images captured by the endoscope through the images displayed in the display device provided on the console to observe real-time status of the surgical site of a patient. The display device may be an original display device of the surgical robot system 1, i.e. the display device provided on the console. The selection interface may be switchable and displayed alternately with the endoscopic image, or displayed together with the endoscopic image. They may be displayed together for example in a vertical arrangement, in a horizontal arrangement, in a way that the selection interface is overlaid on the endoscopic image (as shown in FIG. 6), or the like.

For example, the cursor 324 in the selection interface may be a selection box that surrounds a selection icon 323. When the selection box surrounds a selection icon 323, it is determined that the selection icon 323 is selected. Alternatively, the cursor 324 in the selection interface may also be implemented as highlighting, where the selected selection icon 323 is highlighted or prominently displayed. Alternatively, the cursor 324 in the selection interface may be a mouse cursor as shown in FIG. 6. When the mouse cursor is moved to a selection icon 323, the selection icon 323 is selected. Subsequently, when the surgeon performs a confirmation operation, it is determined that the selection of the selection icon 323 is confirmed.

In some application scenarios, for example, three second robotic arms 210 are provided for auxiliary operations. As shown in FIG. 6, upon the surgeon stepping on the pedal associated with the auxiliary operation, a corresponding switching signal is generated. After receiving the switching signal, the master operation device 300 controls the display device 322 to overlay the selection interface on the endoscope image 325, that is, three corresponding selection icons 323 including a uterine-manipulating-operation selection icon 323a, a retracting-operation selection icon 323b, and an endoscope-holding-operation selection icon 323c are overlaid on the endoscope image 325, these three selection icons correspond to the three second robotic arms 210 that hold the uterine manipulator, the retractor, and the endoscope, respectively. In response to receiving the switching signal, the master operation device 300 further switches the operation mode of the first operation assembly to the third operation mode. The surgeon may manipulate the first operation assembly to move the cursor to perform selection on the selection icons 323. For example, when the cursor stays on the uterine-manipulating-operation selection icon 323a, the uterine-manipulating-operation selection icon 323a is selected. After confirming selection by the surgeon, the master operation device 300 switches the operation mode of the first operation assembly 310 to the second operation mode, so that the first operation assembly 310 is configured to control operation of the second robotic arm 210 corresponding to the uterine-manipulating-operation selection icon 323a, thereby achieving the movement of the uterine manipulator.

The types and numbers of the selection icons 323 are not limited to those illustrated above, and may be determined according to the types of the surgical instruments 500 held by the second robotic arms 210.

In some embodiments, the surgeon may move the cursor using a gripping end of the master controller to select a selection icon.

In some embodiments, the master controller includes at least two joints, and the gripping end is movably coupled to the master operation device 300 through the at least two joints. A respective joint of the at least two joints is provided with an encoder for detecting movement of the respective joint. The master operation device 300 is configured to calculate a movement of the gripping end based on detection of the encoders, and to move the cursor 324 on the selection interface according to the movement of the gripping end.

For example, there are a plurality of master controllers, for example a left-hand master controller and a right-hand master controller, and each master controller has a respective gripping end. Thus, movement of one of the master controllers may be mapped to the movement of the cursor.

In some embodiments, the gripping end includes a rotatable handle. The rotatable handle is provided with an encoder for detecting rotation of the rotatable handle, and the master operation device 300 is configured to switch the selection icon 323 being selected on the selection interface based on detection of the encoder. The detection of the encoder may include detection of the rotation angle and/or rotation direction of the rotatable handle. When the surgeon manipulates the rotatable handle to rotate or twist leftward, the encoder detects the rotation angle and/or leftward rotation information of the rotatable handle, and the selection icon 323 to be selected is switched leftward on the selection interface; or when the surgeon manipulates the rotatable handle to rotate or twist rightward, the selection icon 323 to be selected is switched rightward on the selection interface.

In some embodiments, the surgeon also may manipulate the gripping end to confirm selection on the selection icon.

In some embodiments, the gripping end includes a gripper assembly including a pair of grippers movable relative to each other and a sensor configure to detect movement of the pair of grippers toward or away from each other. The master operation device 300 is configured to confirm selection on a selection icon on the selection interface based on a detection result of the sensor. In some embodiments, the gripper assembly may be implemented as components of the rotatable handle to achieve higher flexibility in controlling surgical instruments. For example, the gripper assembly may be pivotably provided on the gripping end, and the surgeon may manipulate the gripper assembly to rotate to move the cursor to perform selection on a selection icon 323, then may manipulate the gripper assembly to move toward each other to confirm the selection on the selection icon 323. When the master controller is in the state of controlling a surgical instrument (i.e. the first operation mode or the second operation mode), the pair of grippers are configured to control the operations of the end effectors of surgical instruments, such as rotation, opening, and closing. When the master controller is in the state of controlling the cursor 324 in the selection interface (i.e. the third operation mode), the pair of grippers are configured to select and confirm selection on the selection icons 323 on the selection interface.

The structure of the gripping end in details is not limited in the present disclosure, and may have an existing configuration, as more fully described in one of the Chinese Patent Applications CN113375547A, CN113197673A, or CN115486944A, the full disclosure of which is incorporated herein by reference.

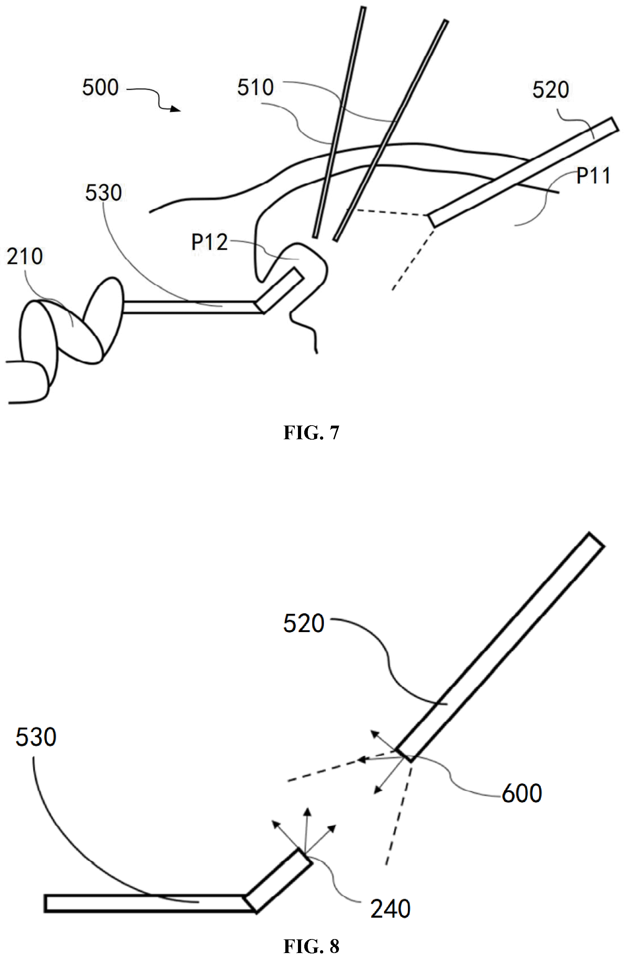

FIG. 7 shows an application scenario of hysterectomy surgery using the surgical robot system according to the present disclosure. The endoscope 520 and an execution instrument 510 enter the abdominal cavity through a small incision in abdomen of patient P1, and the uterine manipulator 530 enters uterus P12 of patient P1 through vagina of patient P1. During the surgery, the endoscope 520 provides the surgeon with a surgical view inside the abdominal cavity P11, and the execution instrument 510 is configured to perform surgical operations on the uterus P12. The end of the uterine manipulator 530 is pressed against the inner wall of the uterus P12, and the posture of the uterus P12 is adjusted by changing the position and orientation of the uterine manipulator 530 to facilitate the surgical operations of the execution instrument 510. In some embodiments, the endoscope 520 and the execution instrument 510 are held by the robotic arm of the laparoscopic surgical robot. For the uterine manipulator 530, manual operation on the uterine manipulator 530 by an additional surgical assistant or manipulation of the uterine manipulator 530 using an auxiliary manipulation robot is usually required, due to the fact that the entry position and operation mode of the uterine manipulator 530 are significantly different from other instruments, and the robotic arm of the laparoscopic surgical robot is difficult to perform the operation of the uterine manipulator 530. Therefore, in the surgical robot system according to the present disclosure, the endoscope 520 and the execution instrument 510 are held by the first robotic arms 110 (not shown) of the first slave operation device 100, and the uterine manipulator 530 is held by the second robotic arm 210 of the second slave operation device 200. The surgeon may manipulate the endoscope 520, the execution instrument 510, and the uterine manipulator 530 through the master operation device 300. In FIG. 7, the first slave operation device 100 may be a laparoscopic surgical robot, and the second slave operation device 200 may be an auxiliary manipulation robot.

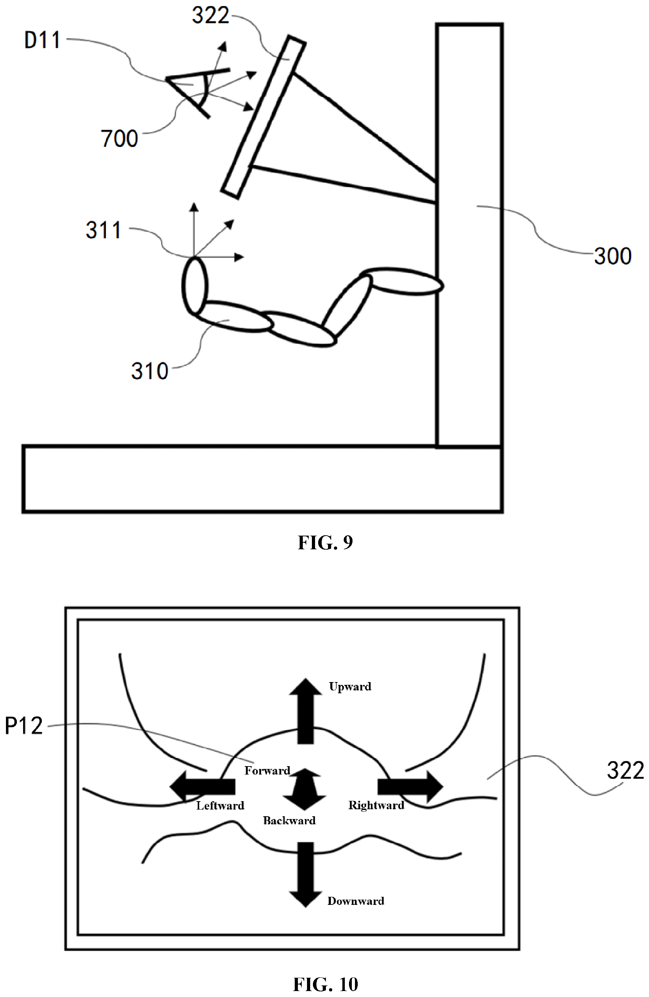

FIG. 8 is a schematic diagram of a reference coordinate system fixed to a surgical instrument 500 and a pose of the surgical instrument of the surgical robot system 1 according to the present disclosure. FIG. 9 is a schematic diagram of an eye D11 of a surgeon, a reference coordinate system fixed to a master controller, and a pose of the master controller of the surgical robot system 1 according to the present disclosure. It is noted that control of the surgical instrument 500 of the auxiliary manipulation robot using the master controller requires coordinate transformation. The homogeneous transformation matrix between coordinate systems may be expressed as:

$$T = \begin{bmatrix} R & D \\ 0,0,0 & 1 \end{bmatrix}$$

where T represents the homogeneous transformation matrix between coordinate systems, R represents a 3×3 rotation matrix, and D represents a 3×1 translation vector.

In the present disclosure, each of the positions of an end of the uterine manipulator 530 in a camera coordinate system 600 is mapped to a respective position of an end of the master controller in an eye coordinate system 700, using the following formula:

$$_u^e D\,' - _u^e D = k \times \left( _m^b D\,' - _m^b D \right)$$

where $(_u^e)D$ represents the translation vector for the homogeneous transformation matrix between a uterine-manipulation-tip coordinate system 240 and the camera coordinate system 600, $(_m^b)D$ represents the translation vector for the homogeneous transformation matrix between a master-controller-tip coordinate system 311 in the eye coordinate system 700 and the camera coordinate system 600, (*)' represents a new position, and k represents a proportional factor.

With the above formula, the linear displacement of the first robotic arm 110 in the eye coordinate system 700 is scaled proportionally and mapped into a linear displacement command for the end of the uterine manipulator 530 in the camera coordinate system 600, thereby generating new position information for the end of the uterine manipulator 530 in the camera coordinate system 600. The new position information is converted into movements of the joints of the auxiliary manipulation robot by a controller of the auxiliary manipulation robot.

FIG. 10 is a schematic diagram of an interface in the display device 322 of the surgical robot system 1 according to the present disclosure. The movements of the master controller by the surgeon correspond to the up, down, left, right, back, and forth movements of the uterine manipulator 530 displayed on the display device 322 (i.e. in the camera coordinate system 600), such that it is more convenient for the surgeon to control the uterine manipulator 530.

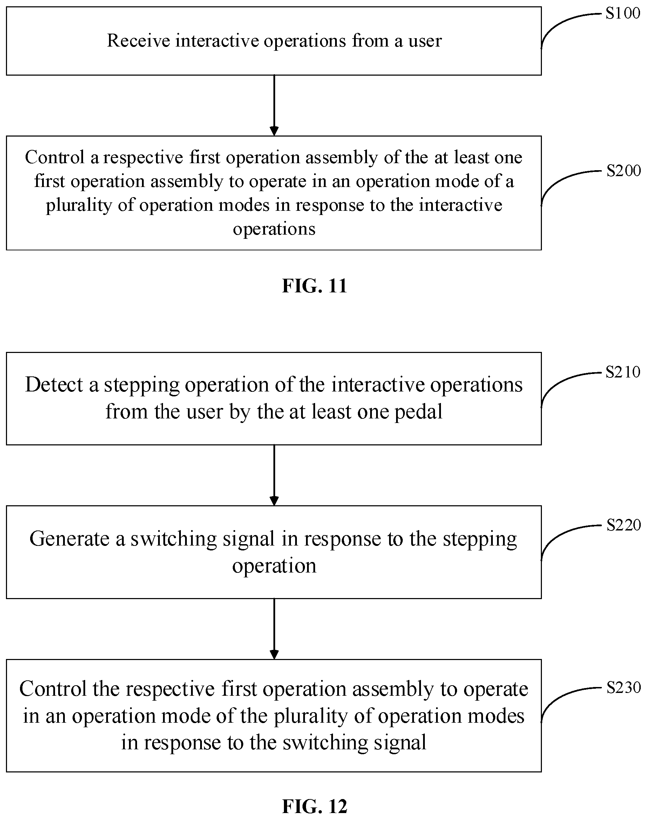

The present disclosure further provides a method for controlling a surgical robot system. FIG. 11 is a flowchart of the method for controlling a surgical robot system according to some embodiments of the present disclosure. The method is applicable to a surgical robot system. The surgical robot system includes a first slave operation device including a first robotic arm, a second slave operation device including a second robotic arm, and a master operation device communicatively coupled to the first slave operation device and the second slave operation device. The master operation device includes at least one first operation assembly. As shown in FIG. 11, the method includes operations S100 and S200.

In operation S100, interactive operations from a user are received.

In operation S200, a respective first operation assembly of the at least one first operation assembly is controlled to operate in an operation mode of a plurality of operation modes in response to the interactive operations.

The plurality of operation modes include a first operation mode and a second operation mode, operation of the first robotic arm is controlled in the first operation mode, and operation of the second robotic arm is controlled in the second operation mode.

In some embodiments, the master operation device further includes a second operation assembly. The operation S100 further includes: receiving, by the second operation assembly, the interactive operations from the user.

FIG. 12 is a flowchart of the operation S200 of the method shown in FIG. 11 according to some embodiments of the present disclosure. In some embodiments, the second operation assembly includes at least one pedal, and the operation S200 includes operations S210, S220, and S230.

In operation S210, a stepping operation of the interactive operations from the user is detected by the at least one pedal.

In operation S220, a switching signal is generated in response to the stepping operation.

In operation S230, the respective first operation assembly is controlled to operate in an operation mode of the plurality of operation modes in response to the switching signal.

In some embodiments, the second slave operation device includes at least two second robotic arms. In some other embodiments, the surgical robot system includes at least two second slave operation devices, and a respective second slave operation device of the at least two second slave operation devices includes at least one second robotic arm.

The second operation assembly includes at least two pedals, and each of the at least two pedals is operatively coupled to a respective second robotic arm. The operation S230 may include: controlling the respective first operation assembly to operate in the second operation mode in response to the switching signal, where operation of the respective second robotic arm is controlled in the second operation mode.

In some embodiments, the second slave operation device includes the at least two second robotic arms. In some other embodiments, the surgical robot system includes at least two second slave operation devices, and a respective second slave operation device of the at least two second slave operation devices includes at least one second robotic arm.

The second operation assembly further includes a display device configured to display a selection interface. The selection interface has at least two selection icons to be selected by the user, and each of the at least two selection icons corresponds to a respective second robotic arm. The plurality of operation modes further include a third operation mode.

The operation S230 may include: controlling the respective first operation assembly to operate in the third operation mode in response to the switching signal, where in the third operation mode, a cursor in the selection interface is controlled to select and confirm selection on a selection icon; controlling the display device to display the selection interface; and controlling the respective first operation assembly to operate in the second operation mode in response to selection on a selection icon being confirmed, where in the second operation mode, operation of a respective second robotic arm associated with the selection icon being selected is controlled.

In some embodiments, the respective first operation assembly includes a gripping end configured to be held by the user. The gripping end is movably coupled to the master operation device through at least two joints, and a respective joint of the at least two joints is provided with an encoder.

The operation of controlling the respective first operation assembly to operate in the third operation mode may include: detecting, by the encoder, movement of the respective joint to obtain a detection result of the encoder for the respective joint; calculating a movement of the gripping end based on the detection result; and moving the cursor on the selection interface according to the movement of the gripping end.

In some embodiments, the respective first operation assembly includes a gripping end configured to be held by the user and including a rotatable handle, and the rotatable handle is provided with an encoder.

The operation of controlling the respective first operation assembly to operate in the third operation mode may include: detecting, by the encoder, rotation of the rotatable handle to obtain a detection result of the encoder; and switching the selection icon being selected on the selection interface based on the detection result of the encoder.

The gripping end includes a gripper assembly including a pair of grippers movable relative to each other and a sensor configure to detect movement of the pair of grippers toward or away from each other.

The operation of controlling the respective first operation assembly to operate in the third operation mode may further include: detecting, by the sensor, the movement of the pair of grippers toward or away from each other; and confirming selection on a selection icon on the selection interface in response to the sensor detecting that the pair of grippers are moved toward each other.

The surgical robot system and the method for controlling the surgical robot system provided in the embodiments of the present disclosure can provide richer surgical angles and operation modes, and provide the surgeon with more degrees of freedom and operability, thereby reducing the need for additional assistants during surgery, simplifying the surgical operation process, and improving surgical efficiency and accuracy. Moreover, by providing additional pedals or selection methods using the display device, switching between slave ends can be achieved, thereby providing the surgeon with a more convenient control method. With the first operation assembly, the surgeon can control the second robotic arm of the second operation device and the cursor on the display device more intuitively, thereby lowering the learning curve of the surgeon.

The method embodiments substantially correspond to the device embodiments, and reference may be made to the device embodiments for relevant information. The method embodiments and the device embodiments complement each other.

The surgical robot system according to the embodiments of the present disclosure further includes a computing device including at least a memory, a processor, and a computer program stored in the memory and executable by the processor. When the processor executes the computer program, operations of the method provided in the embodiments of the present disclosure are performed by the processor. The computing device may be integrated into the master operation device, integrated into the slave operation device, or be provided independently of the master operation device and the slave operation device.

The computing device may include a processor, a memory, input/output interfaces, communication interfaces, and buses. The processor, memory, input/output interfaces, and communication interfaces are interconnected by the buses to achieve inter-communication of the computing device.

The processor may be implemented using a general-purpose central processing unit (CPU), microprocessor, application specific integrated circuit (ASIC), or one or more integrated circuits to execute relevant programs and implement the technical solutions provided in the embodiments of the present disclosure. The processor may further include a graphics card, which may be an Nvidia Titan X graphics card, a 10120Ti graphics card, or the like.

The memory may be implemented in the form of read-only memory (ROM), random access memory (RAM), static storage devices, dynamic storage devices, or the like. The memory can store operating systems and other application programs. When implementing the technical solutions provided in the embodiments of the present disclosure using software or firmware, the relevant program codes are saved in the memory and called and executed by the processor.

The input/output interface is configured to connect input/output modules to achieve information input and output. The input/output/modules may be configured as components in the device or externally connected to the device to provide corresponding functions. The input devices may include a keyboard, a mouse, a touch screen, a microphone, a pedal, a button, various sensors, and the like. The output devices may include a display, a speaker, a vibrator, a indicator light, and the like.

The communication interface is configured to connect the communication module to achieve communication interaction between the device and other devices. The communication module may achieve communication using wired methods (such as USB, Ethernet, or the like) or wireless methods (such as mobile networks, WIFI, Bluetooth, or the like).

The bus includes a pathway for transmitting information between various components (such as the processor, the memory, the input/output interfaces, and the communication interfaces) of the device.

It is noted that although the device is shown to include only the processor, memory, input/output interfaces, communication interfaces, and buses, in practice, the device may further include other components necessary for normal operation. In addition, those skilled in the art shall understand that the above-illustrated device may only include the components necessary for implementing the embodiments of the present disclosure, and do not necessarily include all the components shown in the drawings.

Embodiments of the present disclosure further provide a non-transitory computer-readable storage medium configured to store a computer program, which, when executed by a processor, causes the processor to perform operations of the method provided in the embodiments of the present disclosure.

The computer-readable medium, including permanent and non-permanent computer-readable medium, removable and non-removable computer-readable medium, may be configured to achieve information storage using any method or technology. Information may be computer-readable instructions, data structures, modules of programs, or other data. Examples of the storage medium for computers include, but are not limited to, phase-change random access memory (PRAM), static random access memory (SRAM), dynamic random access memory (DRAM), random access memory (RAM) of other types, read-only memory (ROM), electrically erasable programmable read-only memory (EEPROM), flash memory or other memory technologies, read-only optical disc read-only memory (CD-ROM), digital versatile disc (DVD) or other optical storage, magnetic tape cartridges, magnetic disk storage or other magnetic storage devices, or any other non-transmission medium that may be used to store information accessed by the computing device. According to the definition in the present disclosure, the computer-readable medium does not include transitory computer-readable medium such as modulated data signals and carriers.

From the above illustration, it can be clearly understood by those skilled in the art that the embodiments of the present disclosure may be implemented using software and necessary general hardware platforms. Based on this understanding, the technical solutions according to the embodiments of the present disclosure that essentially contribute to the related technologies may be in the form of a software product. The computer software product may be stored in a storage medium such as ROM/RAM, magnetic disk, optical disk, or the like, and include several instructions to enable a computing device (which may be a personal computer, server, network device, or the like) to perform operations of the method illustrated in the embodiments or parts of the embodiments of the present disclosure.

The above illustration is about only the preferred embodiments of the present disclosure and is not intended to limit the present disclosure. Any modifications, equivalent substitutions, improvements, or the like made within the principles of the present disclosure should be included within the scope of protection of the present disclosure.

## Claims

1. A surgical robot system, comprising:

    a first slave operation device comprising a first robotic arm;
    one or more second slave operation devices comprising one or more second robotic arms; and
    a master operation device communicatively coupled to the first slave operation device and the one or more second slave operation devices;
    wherein the master operation device comprises at least one first operation assembly configured to receive interactive operations from a user to control the first robotic arm or the one or more second robotic arms; and

wherein a respective first operation assembly of the at least one first operation assembly is operable in a first operation mode and a second operation mode, the respective first operation assembly is configured to control operation of the first robotic arm in the first operation mode, and is configured to control operation of the one or more second robotic arms in the second operation mode.

2. The surgical robot system according to claim 1, wherein the master operation device further comprises a second operation assembly configured to receive interactive operations from the user to switch an operation mode of the respective first operation assembly.

3. The surgical robot system according to claim 2, wherein the second operation assembly comprises at least one pedal configured to detect a stepping operation from the user and to generate a switching signal, and the master operation device is configured to switch the operation mode of the respective first operation assembly in response to the switching signal.

4. The surgical robot system according to claim 3, wherein the one or more second slave operation devices comprise at least two second robotic arms, and/or the surgical robot system comprises at least two second slave operation devices, and a respective second slave operation device of the at least two second slave operation devices comprises at least one second robotic arm;

wherein the second operation assembly comprises at least two pedals, and each of the at least two pedals is operatively coupled to a respective second robotic arm and is configured to detect the stepping operation from the user and to generate a respective switching signal; and

wherein the master operation device is configured to switch the operation mode of the respective first operation assembly into the second operation mode in response to the respective switching signal, and the respective first operation assembly is configured to control, in the second operation mode, operation of the respective second robotic arm.

5. The surgical robot system according to claim 3, wherein the one or more second slave operation devices comprise at least two second robotic arms, and/or the surgical robot system comprises at least two second slave operation devices, and a respective second slave operation device of the at least two second slave operation devices comprises at least one second robotic arm;

wherein the second operation assembly further comprises a display device configured to display a selection interface, the selection interface with at least two selection icons to be selected by the user, and each of the at least two selection icons associated to a respective second robotic arm; wherein the respective first operation assembly is further operable in a third operation mode, and in the third operation mode of the respective first operation assembly, the respective first operation assembly is configured to control a cursor in the selection interface to select and confirm selection on a selection icon; wherein the master operation device is configured to switch the operation mode of the respective first operation assembly into the third operation mode in response to the switching signal and control the display device to display the selection interface, and further configured to switch the operation mode of the respective first operation assembly into the second operation mode in response to a selection icon being selected; and wherein in the second operation mode, the respective first operation assembly is configured to control operation of a respective second robotic arm associated with the selection icon being selected.

6. The surgical robot system according to claim 5, wherein the respective first operation assembly comprises a gripping end configured to be held by the user, the gripping end is movably coupled to the master operation device through at least two joints, and a respective joint of the at least two joints is provided with an encoder for detecting movement of the respective joint; and wherein the master operation device is configured to calculate a movement of the gripping end based on detection of the encoder, and to move the cursor on the selection interface according to the movement of the gripping end.

7. The surgical robot system according to claim 5, wherein the respective first operation assembly comprises a gripping end configured to be held by the user and comprising a rotatable handle, the rotatable handle is provided with an encoder for detecting rotation of the rotatable handle, and the master operation device is configured to switch the selection icon being selected on the selection interface based on detection of the encoder.

8. The surgical robot system according to claim 6 or claim 7, wherein the gripping end comprises a gripper assembly comprising a pair of grippers movable relative to each other and a sensor configure to detect movement of the pair of grippers toward or away from each other, and the master operation device is configured to confirm selection on a selection icon on the selection interface in response to the sensor detecting that the pair of grippers are moved toward each other.

9. A method for controlling a surgical robot system, wherein the surgical robot system comprises a first slave operation device comprising a first robotic arm, one or more second slave operation devices comprising one or more second robotic arms, and a master operation device communicatively coupled to the first slave operation device and the one or more second slave operation devices, and the master operation device comprises at least one first operation assembly;

  wherein the method comprises:

    receiving interactive operations from a user; and controlling a respective first operation assembly of the at least one first operation assembly to operate in an operation mode of a plurality of operation modes in response to the interactive operations;
    wherein the plurality of operation modes comprise a first operation mode and a second operation mode, operation of the first robotic arm is controlled in the first operation mode, and operation of the one or more second robotic arms is controlled in the second operation mode.

10. The method according to claim 9, wherein the master operation device further comprises a second operation assembly;
  receiving the interactive operations from the user comprises:
  receiving, by the second operation assembly, the interactive operations from the user.

11. The method according to claim 10, wherein the second operation assembly comprises at least one pedal;
  controlling the respective first operation assembly to operate in an operation mode of the plurality of operation modes in response to the interactive operations comprises:

    detecting, by the at least one pedal, a stepping operation of the interactive operations from the user;
    generating a switching signal in response to the stepping operation; and
    controlling the respective first operation assembly to operate in an operation mode of the plurality of operation modes in response to the switching signal.

12. The method according to claim 11, wherein the one or more second slave operation devices comprise at least two second robotic arms, and/or the surgical robot system comprises at least two second slave operation devices, and a respective second slave operation device of the at least two second slave operation devices comprises at least one second robotic arm;

  wherein the second operation assembly comprises at least two pedals, and each of the at least two pedals is operatively coupled to a respective second robotic arm;
  controlling the respective first operation assembly to operate in an operation mode of the plurality of operation modes in response to the switching signal comprises:

    controlling the respective first operation assembly to operate in the second operation mode in response to the switching signal;
    wherein operation of the respective second robotic arm is controlled in the second operation mode.

13. The method according to claim 11, wherein the one or more second slave operation devices comprise at least two second robotic arms, and/or the surgical robot system comprises at least two second slave operation devices, and a respective second slave operation device of the at least two second slave operation devices comprises at least one second robotic arm;

  wherein the second operation assembly further comprises a display device configured to display a selection interface, the selection interface has at least two selection icons to be selected by the user, and each of the at least two selection icons corresponds to a respective second robotic arm;
  wherein the plurality of operation modes further comprise a third operation mode;
  controlling the respective first operation assembly to operate in an operation mode of the plurality of operation modes in response to the switching signal comprises:

    controlling the respective first operation assembly to operate in the third operation mode in response to the switching signal, wherein in the third operation mode, a cursor in the selection interface is controlled to select and confirm selection on a selection icon;
    controlling the display device to display the selection interface; and

controlling the respective first operation assembly to operate in the second operation mode in response to selection on a selection icon being confirmed, wherein in the second operation mode, operation of a respective second robotic arm associated with the selection icon being selected is controlled.

14. The method according to claim 13, wherein the respective first operation assembly comprises a gripping end configured to be held by the user, the gripping end is movably coupled to the master operation device through at least two joints, and a respective joint of the at least two joints is provided with an encoder;

controlling the respective first operation assembly to operate in the third operation mode comprises:

detecting, by the encoder, movement of the respective joint to obtain a detection result of the encoder for the respective joint;

calculating a movement of the gripping end based on the detection result; and

moving the cursor on the selection interface according to the movement of the gripping end.

15. The method according to claim 13, wherein the respective first operation assembly comprises a gripping end configured to be held by the user and comprising a rotatable handle, and the rotatable handle is provided with an encoder;

controlling the respective first operation assembly to operate in the third operation mode comprises:

detecting, by the encoder, rotation of the rotatable handle to obtain a detection result of the encoder; and

switching the selection icon being selected on the selection interface based on the detection result of the encoder.

16. The method according to claim 14 or claim 15, wherein the gripping end comprises a gripper assembly comprising a pair of grippers movable relative to each other and a sensor configure to detect movement of the pair of grippers toward or away from each other;

controlling the respective first operation assembly to operate in the third operation mode further comprises:

detecting, by the sensor, the movement of the pair of grippers toward or away from each other; and

confirming selection on a selection icon on the selection interface in response to the sensor detecting that the pair of grippers are moved toward each other.

17. A surgical robot system, comprising:

a plurality of slave operation devices; and

a master operation device communicatively coupled to the plurality of slave operation devices, wherein the master operation device is configured to control one or more of the plurality of slave operation devices concurrently.

18. The surgical robot system according to claim 17, wherein the surgical robot system is switchable to one of a plurality of control modes comprising at least one exclusive control mode and at least one non-exclusive control mode; and

wherein in a respective exclusive control mode of the at least one exclusive control mode, the master operation device is configured to exclusively control one slave operation device of the plurality of slave operation devices, and in a respective non-exclusive control mode of the at least one non-exclusive control mode, the master operation device is configured to control one or more of the plurality of slave operation devices.

19. The surgical robot system according to claim 18, wherein the master operation device comprises two first operation assemblies, each of the two first operation assemblies is configured to receive interactive operations from a user to control operation of a respective slave operation device of the plurality of slave operation devices;

wherein in the respective exclusive control mode of the surgical robot system, each of the two first operation assemblies is configured to control operation of an instrument selected from instruments in a same slave operation device; and

wherein in the respective non-exclusive control mode of the surgical robot system, each of the two first operation assemblies is configured to control operation of an instrument selected from instruments in the plurality of slave operation devices.

20. The surgical robot system according to any one of claims 17 to 19, wherein the plurality of slave operation devices comprises at least one single-port laparoscopic robot and at least one single-arm robot; or

the plurality of slave operation devices comprises at least one multi-port laparoscopic robot and at least one single-arm robot.

**FIG. 1**

**FIG. 2**

FIG. 3

FIG. 4

B1

321

| Uterine-manipulating operation | Retracting operation | Endoscope-holding operation |

321a  321b  321c

FIG. 5

FIG. 6

**FIG. 7**

**FIG. 8**

322

D11

700

311

310

300

**FIG. 9**

P12

322

Upward

Forward

Leftward

Rightward

Backward

Downward

**FIG. 10**

Receive interactive operations from a user — S100

Control a respective first operation assembly of the at least one first operation assembly to operate in an operation mode of a plurality of operation modes in response to the interactive operations — S200

**FIG. 11**

Detect a stepping operation of the interactive operations from the user by the at least one pedal — S210

Generate a switching signal in response to the stepping operation — S220

Control the respective first operation assembly to operate in an operation mode of the plurality of operation modes in response to the switching signal — S230

**FIG. 12**

## INTERNATIONAL SEARCH REPORT

| International application No. |
| --- |
| **PCT/CN2024/115380** |

### A. CLASSIFICATION OF SUBJECT MATTER

A61B34/37(2016.01)i

According to International Patent Classification (IPC) or to both national classification and IPC

### B. FIELDS SEARCHED

Minimum documentation searched (classification system followed by classification symbols)

IPC:A61B34, B25J

Documentation searched other than minimum documentation to the extent that such documents are included in the fields searched

Electronic data base consulted during the international search (name of data base and, where practicable, search terms used)

CNTXT: VEN: ENTXTC; ENTXT; CJFD; CNKI: 机器人, 主, 从, 机械臂, 机械手, 机器人臂, 臂, 交互, 控制, 切换, 交换, 互换, 激活, 显示, 光标, 编码, 检测; robot?, mechanical, arm?, manipulator?, alternation, control+, switch+, shift+, interchang+, activat+, display+, cursor, encoder?, detect+, sens+

### C. DOCUMENTS CONSIDERED TO BE RELEVANT

| Category* | Citation of document, with indication, where appropriate, of the relevant passages | Relevant to claim No. |
| --- | --- | --- |
| PX | CN 117379188 A (CORNERSTONE ROBOTICS LIMITED) 12 January 2024 (2024-01-12) claims 1-16, description, paragraphs 78-119, and figures 1-11 | 1-20 |
| PX | CN 117695016 A (SHANGHAI MICROPORT MEDBOT (GROUP) CO., LTD.) 15 March 2024 (2024-03-15) description, paragraphs 42-53, and figures 1-15 | 1-4, 9-12, 17-20 |
| X | CN 114025700 A (AURIS HEALTH, INC.) 08 February 2022 (2022-02-08) description, paragraphs 87 and 130-177, and figures 1-31 | 1-20 |
| X | CN 114376734 A (SHANGHAI MICROPORT MEDBOT (GROUP) CO., LTD.) 22 April 2022 (2022-04-22) description, paragraphs 6-19 and 35-68, and figures 1-16 | 1-20 |
| X | CN 114668513 A (INSTITUTE OF MEDICAL ROBOTS AND INTELLIGENT SYSTEMS, TIANJIN UNIVERSITY) 28 June 2022 (2022-06-28) description, paragraphs 32-33 and 50-76, and figures 1-4 | 1-20 |
| X | WO 2021205178 A2 (CMR SURGICAL LIMITED) 14 October 2021 (2021-10-14) description, paragraphs 80-86, 123-127, and 136-138, and figures 1-9 | 1-20 |

☑ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **07 November 2024** | **05 December 2024** |

| Name and mailing address of the ISA/CN | Authorized officer |
| --- | --- |
| **China National Intellectual Property Administration (ISA/CN)** <br> **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| International application No. |
| --- |
| **PCT/CN2024/115380** |

### C. DOCUMENTS CONSIDERED TO BE RELEVANT

| Category* | Citation of document, with indication, where appropriate, of the relevant passages | Relevant to claim No. |
| --- | --- | --- |
| X | US 2013245375 A1 (C/O JOHN HOPKINS TECHNOLOGY TRANSFER THE JOHNS HOPKINS UNIVERSITY et al.) 19 September 2013 (2013-09-19) description, paragraphs 40-50 and 62-193, and figures 1-29 | 1-20 |
| A | CN 113375547 A (CORNERSTONE ROBOTICS LIMITED) 10 September 2021 (2021-09-10) entire document | 1-20 |
| A | CN 110559083 A (SHENZHEN EDGE MEDICAL CO., LTD.) 13 December 2019 (2019-12-13) entire document | 1-20 |
| A | US 2010234857 A1 (INTUITVE SURGICAL OPERATIONS, INC.) 16 September 2010 (2010-09-16) entire document | 1-20 |

Form PCT/ISA/210 (second sheet) (July 2022)

| INTERNATIONAL SEARCH REPORT | | | | International application No. |
|---|---|---|---|---|
| Information on patent family members | | | | PCT/CN2024/115380 |

| Patent document cited in search report | | | Publication date (day/month/year) | Patent family member(s) | | | Publication date (day/month/year) |
|---|---|---|---|---|---|---|---|
| CN | 117379188 | A | 12 January 2024 | None | | | |
| CN | 117695016 | A | 15 March 2024 | None | | | |
| CN | 114025700 | A | 08 February 2022 | EP | 3989793 | A1 | 04 May 2022 |
| | | | | EP | 3989793 | A4 | 19 July 2023 |
| | | | | WO | 2020264418 | A1 | 30 December 2020 |
| | | | | US | 2024108428 | A1 | 04 April 2024 |
| | | | | US | 2020405420 | A1 | 31 December 2020 |
| | | | | US | 11872007 | B2 | 16 January 2024 |
| CN | 114376734 | A | 22 April 2022 | CN | 114376734 | B | 16 January 2024 |
| CN | 114668513 | A | 28 June 2022 | None | | | |
| WO | 2021205178 | A2 | 14 October 2021 | GB | 202105030 | D0 | 26 May 2021 |
| | | | | GB | 2595048 | A | 17 November 2021 |
| | | | | GB | 2595048 | B | 05 June 2024 |
| | | | | GB | 202005244 | D0 | 20 May 2020 |
| | | | | GB | 2593913 | A | 13 October 2021 |
| | | | | GB | 2593913 | B | 02 October 2024 |
| | | | | WO | 2021205178 | A3 | 11 November 2021 |
| | | | | WO | 2021205178 | A4 | 03 February 2022 |
| | | | | EP | 4132407 | A2 | 15 February 2023 |
| | | | | AU | 2021253760 | A1 | 15 December 2022 |
| | | | | AU | 2021253760 | B2 | 08 February 2024 |
| | | | | US | 2023149105 | A1 | 18 May 2023 |
| US | 2013245375 | A1 | 19 September 2013 | US | 9795446 | B2 | 24 October 2017 |
| | | | | US | 2009036902 | A1 | 05 February 2009 |
| | | | | US | 8398541 | B2 | 19 March 2013 |
| CN | 113375547 | A | 10 September 2021 | CN | 113375547 | B | 01 December 2023 |
| CN | 110559083 | A | 13 December 2019 | EP | 4029467 | A1 | 20 July 2022 |
| | | | | EP | 4029467 | A4 | 20 September 2023 |
| | | | | US | 2022257326 | A1 | 18 August 2022 |
| | | | | WO | 2021047522 | A1 | 18 March 2021 |
| | | | | CN | 110559083 | B | 25 August 2020 |
| US | 2010234857 | A1 | 16 September 2010 | US | 8600551 | B2 | 03 December 2013 |

Form PCT/ISA/210 (patent family annex) (July 2022)

**EP 4 767 987 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202311214615X
- CN 202410918476
- CN 113375547 A
- CN 113197673 A
- CN 115486944 A